# EUROPEAN PATENT SPECIFICATION

(11) **EP 0 477 819 B1**
(45) Date of publication and mention of the grant of the patent: **15.03.1995**
(21) Application number: 91116148.7
(22) Date of filing: 23.09.1991
(51) Int. Cl.: C07D 235/26, C07D 235/28, C07D 235/30, A61K 31/415

(54) **Benzimidazole derivatives, their preparation and use**
Benzimidazolderivate, ihre Herstellung und ihre Verwendung
Dérivés de benzimidazol, leur préparation et leur utilisation

(30) Priority: 24.09.1990 US 586900
(43) Date of publication of application: 01.04.1992
(73) Proprietor: NEUROSEARCH A/S, DK-2600 Glostrup (DK)
(72) Inventor: Olesen, Soren-Peter, DK-2700 Bronshoj (DK); Wätjen, Frank, DK-2730 Herlev (DK)
(74) Representative: Grünecker, Kinkeldey, Stockmair & Schwanhäusser Anwaltssozietät

(56) References cited:
- EP-A- 0 051 827
- EP-A- 0 232 937
- CH-A- 460 028
- DE-A- 2 725 957
- FR-A- 1 561 830
- GB-A- 1 408 408
- US-A- 3 989 709

## Description

The present invention relates to novel benzimidazole derivatives, a method of preparing the same, and a pharmaceutical composition comprising the same as well as to their use for the manufacture of a medicament for the treatment of certain disorders such as hypertension, asthma, ischemica and convulsions.

It is generally well known that opening of potassium (K⁺) channels leads to a hyperpolarization and relaxation of cells. The presently known K⁺ channel openers (cromakalim and pinacidil for example) exert their effect primarily via the ATP sensitive K⁺ channel. They have a high affinity for vascular smooth muscle cells and are thus mostly vasodilators. Recent studies indicate, however, that K⁺ channel openers hyperpolarizing neuronal cells also have anticonvulsive and antiischemic effects in the central nervous system (the CNS) (European Journal of Pharmacology 167, 181-183 (1989), Neuroscience Letters 115, 195-200 (1990), Neuroscience 37(1), 55-60 (1990), The Journal of Pharmacology and Experimental Therapeutics 251(1), 98-104 (1989)). Furthermore recent studies demonstrate that potassium channel openers acting on airways smooth muscle (tracheal smooth muscle) cells will have anti-asthmatic effects (Small et al., K⁺-channel opening as a mechanism for relaxing airways smooth muscle in New Anti-Asthma Drugs (pp 89-94) 1988 Birkhäuser Verlag Basel, and Dr. S.G. Dilly, Cromakalim/Lemakalim, experience in hypertension and nocturnal asthma in Conference Documentation, Potassium Channels '90, The Royal College of Physicians, Dec. 6-7, 1990).

Bianchi et al. in Eur. J. Med. Chem. - Chimica Therapeutica 16(4), 321-326 (1981) discloses benzimidazolin-2-one derivatives having anti-ulcer and anti-secretory activity.

Clark et al. in J. Med. Chem. 21(9), 965-978 (1978) discloses imidazo[4,5-b]pyridin-2-one derivatives having analgesic activity.

EP-A-0,051,827 discloses omega-(2-oxo-benzazolinyl)-alkanoic acid derivatives and certain benzimidazol-2-one derivatives useful in the treatment of asthma, allergy, atereosclerosis and inflammation.

EP-A-0,232,937 discloses that certain piperidin-4-amines, and among them certain N-(2-benzimidazolyl)-4-piperidinamines have anti-allergic properties.

CH-A-460,028 discloses that certain 1-phenyl-3-aminoalkyl-benzimidazol-2-one derivatives possess antidepressive and anti-convulsive properties.

US-A-3,989,709 discloses fused ring benzimidazole derivatives having hypotensive properties.

GB-A-1,408,408 discloses that certain 1-alkyl-2-cyanamino-benzimidazoles having anti-hypertensive activity.

FR-A-1,561,830 discloses that certain 1-(4-alkoxy-phenyl)-benzimidazol-2-one derivatives have interesting pharmacological properties.

DE-A-2,725,957 discloses a method for the preparation of benzimidazol-2-one derivatives.

It is the object of the present invention to provide novel benzimidazole compounds which are useful in the treatment of diseases in mammals, including a human, and especially in the treatment of diseases which can be treated by opening cell membrane potassium channels of such mammals.

According to the present invention, this object is achieved with a compound having the formula 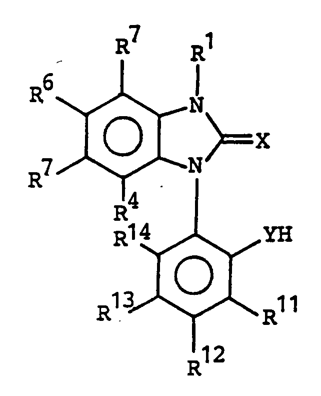
wherein R¹ is hydrogen, NH₂ or C₁₋₆-alkyl which may be branched;
X is 0, S or NCN;
Y is 0 or S;
R⁴, R⁵, R⁶ and R⁷ each independently are hydrogen, halogen, CF₃, NO₂, NH₂, OH, C₁₋₆-alkoxy, C(=O)-phenyl or SO₂NR^{I}R^{II} wherein R^{I} and R^{II} independently are hydrogen or C₁₋₆-alkyl;
R¹¹ is hydrogen, halogen, NO₂ or SO₂NR'R'' wherein R' and R'' independently are hydrogen or C₁₋₆-alkyl;
R¹³ is hydrogen, halogen, phenyl, CF₃ or NO₂;
R¹² is hydrogen or together with R¹³ forms a C₄₋₇-carbocyclic ring which may be aromatic or partially saturated;
R¹⁴ is hydrogen or together with R¹³ forms a C₄₋₇-carbocyclic ring which may be aromatic or partially saturated;
and with a compound as the above which is 5-trifluoromethyl-2,3-dihydro-1-(5-chloro-2-hydroxyphenyl)-1H-2-oxo-benzimidazole,
a compound as the above which is 5-trifluoromethyl-2,3-dihydro-1-(5-phenyl-2-hydroxyphenyl)-1H-2-oxo-benzimidazole,
a compound as the above which is 5-trifluoromethyl-6-nitro-2,3-dihydro-1-(3-nitro-5-chloro-2-hydroxyphenyl)-1H-2-oxo- benzimidazole.

Further, the present invention provides the use of a compound of the above general formula for the manufacture of a medicament for the treatment of a disorder of an animal body, including human, responsive to the opening of potassium channels and, more specifically, to the use for the manufacture of a medicament for treating hypertension, asthma, ischemia or convulsions.

According to the present invention, there is also provided a pharmaceutical composition comprising a therapeutically-effective amount of a compound of the above general formula together with a pharmaceutically-acceptable carrier.

In accordance with the present invention, the compound of the above general formula is prepared by a method which comprises the step of reacting a compound having the formula 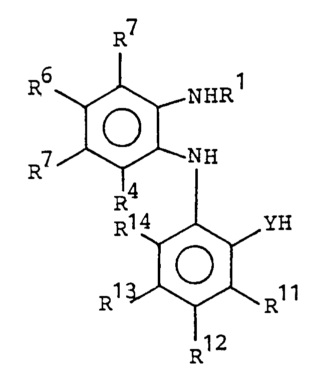
wherein R¹, R⁴, R⁵, R⁶, R⁷, Y, R¹¹, R¹², R¹³ and R¹⁴ have the meanings set forth above with phosgene,
thiocarbonyl diimidazol or N-cyanoimido-S,S'-dimethyldithiocarbonate.

The compounds of the present invention are potent openers of the high conductance, calcium activated K⁺-channel, sometimes referred to as the Big K⁺ channel or the BK_{Ca} channel. The BK_{Ca} channel is present in most neuronal cells, in airway and vascular smooth muscle cells as well as in pancreatic β-cells.

The ability of the compounds of the present invention to open the BK_{Ca} channel can be demonstrated in several ways.

All experiments were performed with patch-clamp technique (Hamill et al., Pflügers Arch. 391, 85-100 (1981). The ion composition of the internal solution was (in mM) 4 NaCl, 140 KCl, 1 CaCl₂, 1 MgCl₂, 2 EGTA, 10 HEPES and the external solution contained 140 NaCl, 4 Kcl, 2 CaCl₂, 1 MgCl₂ and 10 HEPES.

### Whole Cell Recordings

The membrane potential of N1E-115 neuroblastoma cells was determined in whole-cell recordings using current clamp mode (HEKA EPC-9 patch-clamp amplifier). Due to the high-resistance seal and the large size of these cells the recorded membrane potential stayed stable for periods of 30-60 min, although at somewhat depolarized values (-15 - -60 Mv).

Administration of for example 1-(5-chloro-2-hydroxyphenyl)-5-trifluoromethyl-1,3-dihydro-2H-benzimidazol-2-one (30 »M) to the bath hyperpolarized the cells after a delay of 1-3 min. The average hyperpolarization was -14 Mv (SD = -5 Mv, n = 8), and the effect was largest in the cells being most depolarized prior to administration of the compound.

The equilibrium potentials for K⁺, Cl⁻, and Na⁺ in these experiments were -90 mV, 0 mV, and +90 mV, respectively (cf. ion composition above). Thus, since potassium is the only ion having a reversal potential more negative than the resting membrane potential the observed hyperpolarization induced by the test compound must be explained through an increased potassium conductance.

### Single Channel Experiments

In inside-out patches of cerebellar granule cell membrane single BK_{Ca} channels were activated by for example 5-trifluoromethyl-1-(5-chloro-2-hydroxyphenyl)-1,3-dihydro-2H-benzimidazole-2-one (3-10 »M), 5-trifluoromethyl-1-(2-hydroxyphenyl)-1,3-dihydro-2H-benzimidazole-2-one (10-30 mM), 5-trifluoromethyl-1-(5-phenyl-2-hydroxyphenyl)-1,3-dihydro-2H-benzimidazole-2-one (3-30 »M). All compounds increased the open probability of the BK_{Ca} channel with several hundred percent. No effect was found on the delayed rectifier K⁺ channel or on the Cl⁻ channels also present in the patches.

The BK_{Ca} channel in granule cells has been studied in symmetric 144 mM K⁺, and under these conditions the average conductance is 154 pS (76 pS with 4 mM external K⁺). Charybdotoxin blocks the channel in outside-out patches with the characteristic blocking pattern of silent periods interdispersed with periods of low or normal activity, thus confirming the identity of the channel activated by the compounds of the invention.

Likewise in cultured bovine aortic smooth muscle cells in which the BK_{Ca} is the predominant K⁺ channel for example 1,3-dihydro-1-(5-chloro-2-hydroxyphenyl)-5,6-dichloro-2H-benzimidazole-2-one (3-30 »M), 1,3-dihydro-1-(3-hydroxy-2-naphthyl)-5-trifluoromethyl-2H-benzimidazole-2-one (3-30 »M), 5-trifluoromethyl-1-(5-chloro-2-hydroxyphenyl)-1,3-dihydro-2H-benzimidazole-2-one (3-30 »M), 5-trifluoromethyl-1,3-dihydro-1-(2-hydroxy-5-phenylphenyl)-2H-benzimidazole-2-one (3-30 »M), 1,3-dihydro-1-(3-nitro-5-chloro-2-hydroxyphenyl)-5-trifluoromethyl-6-nitro-2H-benzimidazole-2-one (1-3 »M) and 1,3-dihydro-1-(2-hydroxyphenyl)-5-trifluoromethyl-2H-benzimidazole-2-thione (3-30 »M) significantly activated the BK_{Ca} channel.

Likewise in cultured guinea pig tracheal smooth muscle cells in which a 160-180 pS BK_{Ca} channel is expressed for example 1,3-dihydro-1-(5-chloro-2-hydroxyphenyl)-5-trifluoromethyl-2H-benzimidazole-2-one potently activated the BK_{Ca} channel.

The compounds of the invention, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing 10 mg of active ingredients or, more broadly, 0.1 to 100 mg, per tablet, are accordingly suitable representative unit dosage forms.

### Method of Treating

Medicaments comprising the compounds of this invention are extremely useful in the treatment of disorders of mammals due to the potent potassium channel activating properties of the compounds of this invention. Thus, medicaments comprising the compounds of this invention are extremely useful in the treatment of convulsions, asthma, hypertension, ischemia, and other disorders sensitive to potassium channel activating activity such as epilepsia.

### EXAMPLE 1

### Intermediates

a) N-(2-hydroxyphenyl)-2-nitroaniline.
   A solution of 2-nitrofluorobenzene (2 g, 20 mmol), 2-aminophenol (2.2 g, 20 mole) and triethylamine (2.8 ml) in tetrahydrofuran was stirred for 16 h at 100°C and then for 48 h at RT. The reaction mixture was then poured onto a mixture of 50 ml water and 50 ml hexane. The phases were separated and the aqueous phase was extracted with ether. The obtained organic solution was extracted with 20 ml 2 N sodium hydroxide. The aqueous phase was neutralized and acidified with hydrochloric acid. The precipitate was extracted with ether which was dried with MgSO₄. The organic solution was evaporated to give an orange oil which crystallized over a short period. Yield 0.6 g, of the title compound, M.p. 132-134°C. The following compounds were prepared in a similar manner.
   N-(2-hydroxyphenyl)-2-nitro-4,6-dichloro-aniline, M.p. 182-184°C, by reaction between 2,3,5-trichloro-nitrobenzene and 2-aminophenol.
   N-(2-hydroxyphenyl)-2-nitro-4-trifluoromethyl-aniline, M.p. oil at RT, by reaction between 2-aminophenol and 2-chloro-5-trifluoromethyl-nitrobenzene.
   N-(2-methoxy-5-chloro-phenyl)-2-nitroaniline, M.p. 107-108°C, by reaction between 2-fluoro-nitrobenzene and 2-methoxy-5-chloro-aniline.
   N-(2-methoxy-5-chloro-phenyl)-4-trifluoromethyl-2-nitroaniline, M.p. 133-136°C, by reaction between 2-chloro-5-trifluoromethyl-nitrobenzene and 2-methoxy-5-chloro-aniline.
   N-(2-methoxy-5-chlorophenyl)-2-nitro-4,6-dichloroaniline, M.p. 60-61°C, by reaction between 2,3,5-trichloro-nitrobenzene and 2-methoxy-5-chloro-aniline.
   N-(2-methoxyphenyl)-2-nitroaniline, M.p. 80-82°C by reaction between 2-methoxyaniline and 2-fluoro-nitrobenzene.
b) N-(2-methoxy-5-phenylphenyl)-2-nitroaniline.
   To a stirred solution of 2-fluoronitrobenzene (10 mmol, 1 ml) and 2-methoxy-5-phenylaniline (10 mmol, 2.0 g) in tetrahydrofuran (50 ml) was added sodium hydride (10 mmol, 380 mg - 60% in mineral oil). The reaction mixture was stirred at reflux temperature for 8 h and then evaporated in vacuo. The residue was partitioned between ether (100 ml) and 1N hydrochloric acid (50 ml). The organic phase was dried and evaporated in vacuo. The residue was extracted several times at room temperature with petrol ether, in order to remove remaining 2-fluoronitrobenzene. This treatment left the title compound as a dark yellow oil.
   The following compounds were prepared in a similar manner.
   N-(2-methoxyphenyl)-2-nitro-4-trifluoromethyl aniline, M.p. 112-114°C by reaction between o-anisidine and 4-chloro-3-nitro-benzotrifluoride.
   N-(2-methoxy-5-trifluoromethylphenyl)-2-nitro-4-trifluoromethyl aniline, M.p. 90-95°C by reaction between 3-amino-4-methoxybenzotrifluoride and 4-chloro-3-nitrobenzotrifluoride.
   N-(2-methoxyphenyl)-2-nitro-4-dimethylsulfamoyl-aniline, M.p. 136-138°C, by reaction between 2-methoxy-aniline and 5-dimethylsulfamoyl-2-chloro-nitrobenzene.
   N-(2-methoxyphenyl)-2-nitro-4,5-dichloroaniline, M.p. 145-148°C, by reaction between 2-methoxyaniline and 2-fluoro-4,5-dichloronitrobenzene.
   N-(2-hydroxy-1-naphthyl)-2-nitro-4-trifluoromethyl-aniline, M.p. 187-190°C, by reaction between 2-chloro-5-trifluoromethyl-nitrobenzene and 2-hydroxy-1-amino-naphthalene.
   N-(5-chloro-2-methoxyphenyl)-2-nitro-4-methoxyaniline, M.p. 112-114°C, by reaction between 2-methoxy-5-chloroaniline and 2-nitro-4-methoxychlorobenzene.
   N-(2-methoxyphenyl)-2-nitro-4-trifluoromethyl-5-chloroaniline, M.p. 142-145°C, by reaction between 2-methoxyaniline and 2-chloro-4-chloro-5-trifluoromethyl-nitrobenzene.
c) 4,5-dichloro-2-nitro-N-(2-hydroxyphenyl)aniline, M.p. 193-194°C.
   A solution of 4,5-dichloro-2-fluoro-nitrobenzene (2.1 g, 10 mmol) and o-aminophenol (2.1 g, 19 mmol) in a mixture of dimethylformamide (25 ml) and pyridine was stirred overnight at 60°C. The solvents were evaporated, and the residue was partitioned between ether (100 ml)/1N Hcl (100 ml). The organic phase was washed with water, dried and evaporated. This gave a crystalline residue of the crude product, which was recrystallized in methanol, M.p. 193-194°C.
   In a similar manner the following compound was prepared:
   2-nitro-4-dimethylsulfamoyl-N-(5-chloro-2-hydroxyphenyl)-aniline, oil at RT, by reacting 2-chloro-5-dimethylsulfamoyl-nitrobenzene and 2-amino-4-chloro-phenol.

### EXAMPLE 2

### Intermediates

a) 2-benzoxazolinone
   1.09 g 2-aminophenol was dissolved in dry tetrahydrofuran (25 ml) and triethylamine (3.09 g). Then phosgene (5.5 ml) was added dropwise and the resulting mixture was refluxed for 4 h and was left at RT overnight. The reaction mixture was filtrated and the filtrate was evaporated. Yield 0.75 g of title compound, M.p. 137-138°C.
   The following compounds were prepared in a similar manner.
   5-chloro-2-benzoxazolinone, M.p. 190-192°C.
   5-nitro-2-benzoxazolinone, M.p. 207-210°C.
b) N-(2-nitrophenyl)-2-benzoxazolinone
   0.7 g of 2-benzoxazolinone was dissolved in 5 ml of dimethylformamide and 0.52 ml 2-fluoro-nitrobenzene was added. Then 0.27 g sodium hydride (60% oil suspension) was added. The mixture was stirred at RT until gas development ceased and thereafter at 80°C for 1 h. The reaction mixture was cooled and poured unto 25 ml water and 1 ml glacial acetic acid. The precipitate was filtered off and was washed with water and diethyl ether. Yield 1.0 g of the title compound, M.p. 153-155°C.
   The following compounds were prepared in a similar manner.
   N-(2-nitro-4-trifluoromethylphenyl)-5-chloro-2-benzoxazolinone, M.p. 177-179°C by reaction between 2-chloro-5-trifluoro-nitrobenzene and 5-chloro-2-benzoxazolinone.
   N-(2-nitrophenyl)-2-benzothiazolinone, M.p. 92-95°C by reaction between 2-fluoronitrobenzene and 2-benzothiazolinone.
   N-(2-nitro-4-fluorophenyl)-5-chloro-2-benzoxazolinone by reaction between 2,4-difluoro-nitrobenzene and 5-chloro-2-benzoxazolinone. The product was used without further purification.
   N-(2-nitro-4-trifluoromethylphenyl)-5-phenyl-*o*-anisidine by reaction between 2-fluoro-5-trifluoromethyl-nitrobenzene and 5-phenyl-*o*-anisidine. The product was used without further purification.
   N-(2-nitro-4-trifluoromethyl-5-chlorophenyl)-5-phenyl-*o*-anisidine by reaction between 2-chloro-4-chloro-5-trifluoromethyl-nitrobenzene and 5-phenyl-*o*-anisidine. The product was used without further purification.
   N-(2-nitrophenyl)-3,5-dichloro-2-methoxy-aniline by reaction between 2-fluoro-nitrobenzene and 3,5-dichloro-*o*-anisidine, brown crystals, M.p. 104-105°C.
   N-(2-nitro-4-benzoylphenyl)-5-chloro-2-benzoxazolinone by reaction between 4-chloro-3-nitrobenzophenone and 5-chloro-2-benzoxazolinone. The product was used without further purification.
   N-(2,4-dinitrophenyl)-5-chloro-2-benzoxazolinone by reaction between 2,4-dinitro-chlorobenzene and 5-chloro-2-benzoxazolinone. The product was used without further purification.
c) N-(2-hydroxyphenyl)-2-nitro-aniline
   0.23 g N-(2-nitrophenyl)-2-benzoxazolinone was heated at 70°C for 1 h in a mixture of 2 ml 4N sodium hydroxide and 5 ml 96% ethanol. The reaction mixture was then cooled and neutralized with hydrochloric acid. The red precipitate was isolated by filtration or extraction with diethyl ether followed by evaporation. Yield 180 mg title compound, M.p. 133-135°C.
   The following compounds were prepared in a similar manner.
   2-nitro-N-(5-chloro-2-hydroxyphenyl)-4-trifluoromethyl aniline, M.p. 67-70°C from N-(2-nitro-4-trifluoromethylphenyl)-5-chloro-2-benzoxazolinone.
   N-(2-mercaptophenyl)-2-nitroaniline, M.p. 105-110°C from N-(2-nitrophenyl)-2-benzothiazolinone.
   N-(5-chloro-2-hydroxyphenyl)-4-fluoro-2-nitro-aniline from N-(2-nitro-4-fluorophenyl)-5-chloro-2-benzoxazolinone. The product was used without further purification.
   N-(5-chloro-2-hydroxyphenyl)-4-benzoyl-2-nitro-aniline from N-(2-nitro-4-benzoylphenyl)-5-chloro-2-benzoxazolinone. The product was used without further purification.
   N-(5-chloro-2-hydroxyphenyl)-2,4-dinitroaniline from N-(2,4-dinitrophenyl)-5-chloro-2-benzoxazolinone. The product was used without further purification.

### EXAMPLE 3

### Intermediates

2-amino-N-(5-chloro-2-hydroxyphenyl)-4-nitro-anilinehydrochloride.

A mixture of N-(5-chloro-2-hydroxyphenyl)-2,4-dinitroaniline (7.9 g, 25.6 mmol), ammoniumchloride (5.2 g, 98 mmol) and sodiumsulfide nonahydrate (23.5 g, 98 mmol) in methanol (350 ml) was refluxed for 1 h. The reaction was filtered after cooling to room temperature and the filtrate was concentrated in vacuo. The remanecens was dissolved in ethanol, acidified with concentrated hydrochloric acid and the mixture was concentrated in vacuo. Finally the crude product was triturated with diethyl ether yielding the title compound as red/brown crystals. The product was used without further purification.

### EXAMPLE 4

### Intermediates

N-(2-hydroxyphenyl)-2-phenylene diamine hydrochloride.

1.5 g of N-(2-hydroxyphenyl)-2-nitroaniline was hydrogenated under standard conditions, 98 x 10³ Pa (1 atm) with 0.5 g 5% Pd/C as catalyst, ethanol (100 ml) as solvent and 0.6 ml concentrated hydrochloric acid to give the title compound as an oil which crystallized over a short period. Yield: 1.1 g greenish crystals of the title compound, M.p. 220-223°C.

The following compounds were prepared in a similar manner. In cases where the free bases were isolated, addition of hydrochloric acid was omitted during the hydrogenation.

N-(2-methoxyphenyl)-2-amino-4-trifluoroaniline, hydrochloride, M.p. 258-260°C.

2-amino-N-(2-hydroxyphenyl)-4-trifluoromethyl aniline, M.p. 108-109°C.

2-amino-N-(2-methoxy-5-chloro-phenyl)-aniline, M.p. oil at RT. Raney Nickel was used as catalyst.

2-amino-4-trifluoromethyl-N-(2-methoxy-5-chloro-phenyl)-aniline, M.p. oil at RT. Raney nickel was used as catalyst.

2-amino-N-(2-methoxyphenyl)-aniline, M.p. Oil at RT.

2-amino-4,6-dichloro-N-(2-methoxy-5-chlorophenyl)-aniline, M.p. Oil at RT. Raney Nickel was used as catalyst.

2-amino-N-(5-chloro-2-hydroxyphenyl)-4-trifluoromethyl anilinehydrochloride, M.p. 192-195°C. Raney Nickel was used as catalyst.

N-(2-methoxy-5-phenylphenyl)-2-amino aniline, hydrochloride, oil at RT.

N-(2-hydroxyphenyl)-4,5-dichloro-2-amino aniline, M.p. 132-133°C. Raney nickel was used as catalyst.

N-(2-mercaptophenyl)-2-amino aniline, oil at RT.

N-(5-chloro-2-methoxyphenyl)-2-amino aniline. Raney nickel was used as catalyst, oil at RT.

N-(5-chloro-2-methoxy -2-amino-4-methoxy-aniline, M.p. oil at RT. Raney nickel was used as catalyst.

N-(5-chloro-2-hydroxyphenyl)-2-amino-4-dimethylsulfamoyl-aniline, M.p. 207-209°C. Raney nickel was used as catalyst.

N-(2-methoxyphenyl)-2-amino-4-dimethylsulfamoyl-aniline, M.p. 162-164°C. 5% Pd/C was used as catalyst.

N-(2-methoxyphenyl)-2-amino-4-trifluoromethyl-5-chloro-aniline, M.p. 188-190°C. Raney nickel was used as catalyst.

N-(2-methoxyphenyl)-2-amino-4,5-dichloro-aniline, hydrochloride, M.p. 176-178°C. Raney nickel was used as catalyst.

N-(3-hydroxy-2-naphthyl)-2-amino-4-trifluoromethyl-aniline, M.p. 280°C (decomp.). 5% Pd/C was used as catalyst.

N-(2-hydroxy-1-naphthyl)-2-amino-4-trifluoromethyl-aniline, M.p. 154-157°C. 5% Pd/C was used as catalyst.

2-amino-N-(5-chloro-2-hydroxyphenyl)-4-fluoro-anilinehydrochloride, Raney Nickel was used as catalyst. The product was used without further purification.

2-amino-N-(5-phenyl-2-methoxyphenyl)-4-trifluoromethyl-aniline, 5% Pd/C was used as catalyst. The product was used without further purification.

2-amino-N-(5-phenyl-2-methoxyphenyl)-5-chloro-4-trifluoromethyl-aniline, Raney Nickel was used as catalyst. The product was used without further purification.

2-amino-N-(3,5-dichloro-2-methoxyphenyl)-aniline, hydrochloride, Raney Nickel was used as catalyst. Grey crystals, M.p. 191-192°C.

2-amino-N-(5-chloro-2-hydroxyphenyl)-4-benzoyl-aniline, Raney Nickel was used as catalyst. The product was used without further purification.

### EXAMPLE 5

### Intermediates

1,3-dihydro-1-(5-chloro-2-methoxyphenyl)-5-trifluoromethyl-2-cyanoimido-benzimidazole.

N-(5-chloro-2-methoxyphenyl)-2-amino-4-trifluoromethyl-aniline, hydrochloride (1.06 g, 3 mmol), N-cyanoimido-S,S'-dimethyl-dithiocarbonate (0.44 g, 3 mmol) and 60% sodiumhydride in oil (0.24 g, 6 mmol) were mixed in dry tetrahydrofuran (15 ml) and stirred at RT. The reaction mixture was evaporated and dimethylformamide (10 ml) was added to the residue and the resulting mixture was heated at 80°C for 24 h, whereafter 6 mmol additional sodium hydride was added and heating was continued for 24 h. Thereafter water and light petroleum were added. The precipitate was filtrated off and was redissolved in ether. The solution was evaporated and the residue was treated with light petroleum and thereafter with toluene. Yield: 100 mg of the title compound, M.p. 218-220°C.

### EXAMPLE 6

### Intermediates

a) N,N'-di-(2-methoxyphenyl)-urea.
   Under stirring on an ice bath phosgene (20.7 ml, 1.93 M sol in toluene, 40 mmol) was added to a solution of *o*-anisidine (12.3 g, 100 mmol) and triethylamine (11 ml, 80 mmol) in absolute toluene (100 ml) and the mixture was stirred at 90°C for 3 h. After cooling of the mixture to ambient temperature it was poured onto water (100 ml) and the title compound was collected by filtration and used without further purification.
b) 1,3-dihydro-1-(2-methoxyphenyl)-4-methoxy-2H-benzimidazol-2-one
   To a mixture of N,N'-di-(2-methoxyphenyl)-urea (6.81 g, 25 mmol) and tetra-n-butyl-ammoniumbromide (0.4 g, 1.3 mmol) in methylene chloride methanol (1:1 , 200 ml) a solution of sodium hydroxide (2 g in 10 ml water) followed by a solution of sodiumhypochlorit (55 ml, Aldrich) was added at room temperature under vigorous stirring. The mixture was stirred for 4 h and 30 ml water was added. The organic phase was washed with 10 ml 1M hydrogen chloride sol., dried and concentrated in vacuo. Upon trituration with diethyl ether dark grey crystals separated. The crude product was recrystallized twice first from ethanol and then from ethyl acetate yielding the title compound as white crystals, M.p. 246-247°C.

### EXAMPLE 7

### Compounds of invention and Intermediates

1,3-dihydro-1-(2-hydroxyphenyl)-2H-benzimidazol-2-one. Compound of Invention.

2-amino-N-(2-hydroxyphenyl)-aniline (1 g, 4.2 mmol) and 2.1 ml (15 mmol) triethylamine were dissolved in dry tetrahydrofuran (50 ml). Then 2.5 ml (5 mM in toluene) of phosgene was added dropwise, whereafter the mixture was refluxed for 2 h. The resulting mixture was evaporated in vacuo and the residue was partitioned between water and ethyl acetate. The organic phase was extracted with a 1 N sodium hydroxide solution (20 ml). The aqueous phase was neutralized whereafter the crude product was isolated by filtration. The product was recrystallized from 15 ml isopropanol. Yield: 0.5 g of the title compound, M.p. 216-217 mg.

In a similar way the following compounds were synthesized.

5-trifluoromethyl-1,3-dihydro-1-(2-hydroxyphenyl)-2H-benzimidazol-2-one, M.p. 154-155°C. Compound of Invention.

1-(5-chloro-2-hydroxyphenyl)-5-trifluoromethyl-1,3-dihydro-2H-benzimidazol-2-one, M.p. 223-224°C. Compound of Invention.

1,3-dihydro-1-(2-methoxyphenyl)-2H-benzimidazol-2-one, M.p. 184-186°C. Intermediate.

1-(5-chloro-2-methoxyphenyl)-5-trifluoromethyl-1,3-dihydro-2H-benzimidazol-2-one, M.p. 135-138°C. Intermediate.

1-(5-chloro-2-methoxyphenyl)-1,3-dihydro-2H-benzimidazol-2-one, M.p. 168-170°C. Intermediate.

1-(5-chloro-2-methoxyphenyl)-5,7-dichloro-1,3-dihydro-2H-benzimidazol-2-one, M.p. 218-220°C. Intermediate.

1,3-dihydro-1-(2-methoxyphenyl)-5-trifluoromethyl-2H-benzimidazol-2-one, M.p. 223-226°C. Intermediate.

1,3-dihydro-1-(2-mercaptophenyl)-2H-benzimidazol-2-one, M.p. 190-195°C. Compound of Invention.

1,3-dihydro-1-(2-methoxy-5-phenylphenyl)-2H-benzimidazol-2-one, M.p. rearranges at 115°C melts at 185°C. Intermediate.

5,6-dichloro-1,3-dihydro-1-(2-hydroxyphenyl)-2H-benzimidazol-2-one, M.p. 282-285°C. Compound of Invention.

1,3-dihydro-(5-chloro-2-methoxyphenyl)-5-methoxy-2H-benzimidazol-2-one, M.p. 199-202°C. Intermediate.

1,3-dihydro-1-(5-chloro-2-hydroxyphenyl)-5-dimethylsulfamoyl-2H-benzimidazol-2-one, M.p. 100-105°C (decomp.). Compound of Invention.

1,3-dihydro-1-(2-methoxyphenyl)-5-dimethylsulfamoyl-2H-benzimidazol-2-one, M.p. 200-203°C. Intermediate.

1,3-dihydro-1-(2-methoxyphenyl)-5-trifluoromethyl-6-chloro-2H-benzimidazol-2-one, M.p. 289-291°C. Intermediate.

1,3-dihydro-1-(2-methoxyphenyl)-5,6-dichloro-2H-benzimidazol-2-one, M.p. 262-264°C. Intermediate.

1,3-dihydro-1-(3-hydroxy-2-naphthyl)-5-trifluoromethyl-2H-benzimidazol-2-one, M.p. 290-293°C. Compound of Invention.

1,3-dihydro-1-(2-hydroxy-1-naphthyl)-5-trifluoromethyl-2H-imidazole-2-one, M.p. 223-228°C. Compound of Invention.

### EXAMPLE 8

### Compounds of Invention and Intermediates

1,3-dihydro-1-(5-chloro-2-hydroxyphenyl)-5-trifluoromethyl-2H-benzimidazol-2-thione. Compound of Invention.

2-amino-N-(5-chloro-2-hydroxyphenyl)-4-trifluoromethyl-aniline (0.51 g, 1.5 mmol), triethylamine (0.28 ml, 2 mmol) and thiocarbonyl diimidazol (0.36 g, 2 mmol) were dissolved in dry tetrahydrofuran (15 ml) and the mixture was stirred for 30 min. The reaction mixture was evaporated in vacuo.The residue was redissolved in ethanol and water and light petroleum were added. The precipitate was isolated and was purified by column chromatography on silica gel with ether/light petroleum (2:1) as eluent. Yield of title compound was 200 mg, M.p. 248-250°C.

The following compounds were prepared in a similar manner.

1,3-dihydro-1-(5-chloro-2-methoxyphenyl)-5-trifluoromethyl-2H-benzimidazol-2-thione, M.p. 168-170°C. Intermediate.

1,3-dihydro-1-(3-hydroxy-2-naphthyl)-5-trifluoromethyl-2H-benzimidazol-2-thione, M.p. 216-220°C. Compound of Invention.

### EXAMPLE 9

### Compounds of Invention and Intermediates

1,3-dihydro-1-(5-chloro-2-hydroxyphenyl)-5-fluoro-2H-benzimidazol-2-one. Compound of Invention.

A mixture of 2-amino-N-(5-chloro-2-hydroxyphenyl)-5-fluoroanilinehydrochloride (14 mmol) and urea (1.2 g, 20 mmol) in 50 ml absolute dimethylethyleneglycol was stirred at 170° C for 4 h. After cooling to ambient temperature the mixture was poured onto 200 ml ice water and a crystalline product was isolated by filtration. The crude product was treated with active coal in ethanol and recrystallized from toluene to yield the title compound as white crystals, M.p. 256-260°C.

The following compounds were prepared in a similar manner.

1,3-dihydro-1-(5-phenyl-2-methoxyphenyl)-5-trifluoromethyl-2H-benzimidazol-2-one, white crystals after column chromatography using methylene chloride:methanol (9:1) as eluent, M.p. 258-259°C. Intermediate.

1,3-dihydro-1-(5-phenyl-2-methoxyphenyl)-5-trifluoromethyl-6-chloro-2H-benzimidazol-2-one, white crystals, M.p. 295-298°C. Intermediate.

1,3-dihydro-1-(5-chloro-2-hydroxyphenyl)-5-benzoyl-2H-benzimidazol-2-one, White crystals, M.p. 279-280°C. Compound of Invention.

### EXAMPLE 10

### Intermediates

2-ethoxycarbonylamino-N-(3,5-dichloro-2-methoxyphenyl)-aniline. Intermediate.

To a mixture of 2-Amino-N-(3,5-dichloro-2-methoxyphenyl)-aniline (2.23 g, 6.3 mmol) and triethylamine (1.74 ml 12.5 mmol) in absolute methylene chloride (40 ml) ethyl chloroformate (1.2 ml, 13.8 mmol) was added at room temperature and the mixture was refluxed over night. The reaction mixture was allowed to reach room temperature and was diluted with water (40 ml) and the water phase was extracted twice with diethylether. The combined organic phases were dried and concentrated in vacuo yielding a red/brown oil which was used without further purification.

The following compounds were prepared in a similar manner.

2-ethoxycarbonylamino-N-(5-chloro-2-hydroxyphenyl)-4-nitroaniline, brown crystals. The product was used without further purification. Intermediate.

### EXAMPLE 11

### Compounds of Invention and Intermediates

1,3-dihydro-1-(3,5-dichloro-2-methoxyphenyl)-2H-benzimidazol-2-one. Intermediate.

To a solution of 2-ethoxycarbonylamino-N-(3,5-dichloro-2-methoxyphenyl)-aniline (1.94 g, 5.4 mmol) in absolute ethanol (20 ml) small pieces of sodium (0.25 g, 10.8 mmol) were added at room temperature. The mixture was heated at reflux for 1 *1/2* h after all the sodium had reacted. After cooling to room temperature the mixture was poured onto ice water (50 ml) and collected by filtration yielding the title compound as slightly pink crystals, M.p. 202-203°C.

The following compound was prepared in a similar manner.

1,3-dihydro-1-(5-chloro-2-hydroxyphenyl)-2H-5-nitrobenzimidazole-2-one, beige crystals, M.p. 238-239°C. Compound of Invention.

### EXAMPLE 12

### Compounds of Invention

1-(5-chloro-2-hydroxyphenyl)-1,3-dihydro-2H-benzimidazol-2-one
1-(5-chloro-2-methoxyphenyl)-1,3-dihydro-2H-benzimidazol-2-one (0.3 g) was refluxed for 32 h in 48% HBr (1 ml) in acetic acid (5 ml). The reaction mixture was then cooled and 25 ml water was added, whereby the crude product precipitated. To the mixture was added 5 ml diethylether whereafter it was filtered to give 150 mg of the title compound, M.p. 248-250°C.

The following compounds were prepared in a similar manner from the corresponding alkoxy analogues:
1,3-dihydro-1-(2-hydroxy-5-phenylphenyl)-2H-benzimidazol-2-one, M.p. 130°C decomp.

5,7-dichloro-1-(5-chloro-2-hydroxyphenyl)-1,3-dihydro-2H-benzimidazol-2-one, M.p. 206-208°C.

### EXAMPLE 13

### Compound of Invention

1,3-dihydro-1-(2-hydroxyphenyl)-4-hydroxy-2H-benzimidazol-2-one
To a slurry of 1,3-dihydro-1-(2-methoxyphenyl)-4-methoxy-2H-benzimidazol-2-one (500 mg, 1.9 mmol) in 15 mol absolute methylene chloride boron tribromide (350 »l, 3.7 mmol) was added under a nitrogen atmosphere at -78°C and the mixture was allowed to reach room temperature. After stirring for 5 h the mixture was poured onto 150 ml water and was stirred vigorously for 15 min. The phases were separated, the water phase was extracted twice with 50 ml methylene chloride and the combined organic phases were dried and concentrated in vacuo. The crude crystalline product was triturated with a small amount of ethyl acetate yielding the title compound as slightly brown crystals, M.p. 235°C (dec.).

The following compounds were prepared in a similar manner.

1,3-dihydro-1-(5-phenyl-2-hydroxyphenyl)-5-trifluoromethyl-2H-benzimidazol-2-one, white crystals M.p. 258-259°C.

1,3-dihydro-1-(5-phenyl-2-hydroxyphenyl)-5-trifluoromethyl-6-chloro-2H-benzimidazol-2-one, white crystals M.p. 275-277°C.

1,3-dihydro-1-(3,5-dichloro-2-hydroxyphenyl)-2H-benzimidazol-2-one, light grey crystals, M.p. 238-239°C.

### EXAMPLE 14

### Compounds of Invention

1,3-dihydro-1-(5-chloro-2-hydroxyphenyl)-3-methyl-5-fluoro-2H-benzimidazol-2-one.

A mixture of 1,3-dihydro-1-(5-chloro-2-hydroxyphenyl)-5-fluoro-2H-benzimidazol-2-one (300 mg, 1.1 mmol), anhydrous potassium carbonate (415 mg) and iodomethane (68 »l) in 10 ml absolute acetone was refluxed for 5 h. The mixture was filtered, concentrated in vacuo and the crude product was subjected to column chromatography using methylene chloride:acetone (20:1) as eluent. The fractions containing the product was concentrated in vacuo and the title compound was obtained as white crystals, M.p. 247-250°C.

The following compound was prepared in a similar manner.

1,3-dihydro-1-(5-chloro-2-hydroxyphenyl)-3-methyl-5-trifluoromethyl-2H-benzimidazol-2-one, white crystals, M.p. 257-260°C.

### EXAMPLE 15

### Compounds of Invention

1,3-dihydro-1-(5-chloro-3-bromo-2-hydroxyphenyl)-5-trifluoromethyl-2H-benzimidazol-2-one.

To a solution of 1,3-dihydro-1-(5-chloro-2-hydroxyphenyl)-5-trifluoromethyl-2H-benzimidazol-2-one (0.2 g, 0.6 mmol) in glacial acetic acid (5 ml) bromine (45 »l, 0.9 mmol) was added at room temperature and after stirring for 20 min the mixture was poured onto water (25 ml). The crude product was collected by filtration, taken up in hot ethanol and precipitated by the addition of water yielding the title compound as white crystals, M.p. 254-255°C.

### EXAMPLE 16

### Compounds of Invention

1,3-dihydro-1-(5-chloro-3-bromo-2-hydroxyphenyl)-5-trifluoromethyl-6-nitro-2H-benzimidazol-2-one.

To a slurry of 1,3-dihydro-1-(5-chloro-3-bromo-2-hydroxyphenyl)-5-trifluoromethyl-2H-benzimidazol-2-one (1 g, 2.5 mmol) in glacial acetic acid (5 ml) acetic anhydride (2.5 ml) and two drops of concentrated sulfuric acid were added under stirring on an ice bath. When the starting material had gone into solution, an ice cold solution of potassium nitrate (0.3 g, 3 mmol) in concentrated sulfuric acid (1 ml) was added, and the mixture was stirred for 15 min. The reaction mixture was poured onto water, the crystalline product was collected by filtration and the acetate was hydrolysed by reflux in ethanol (50 ml) and 4M sodium hydroxide (5 ml) for 1 h. The reaction mixture was acidified by addition of 4M hydrochloric acid and water was added until precipitation started. The mixture was cooled and the crystals were collected by filtration yielding the title compound as slightly yellow crystals, M.p. 262-264°C.

### EXAMPLE 17

### Compounds of Invention

1,3-dihydro-1-(5-chloro-3-nitro-2-hydroxyphenyl)-5-trifluoromethyl-2H-benzimidazol-2-one.

To a solution of 1,3-dihydro-1-(5-chloro-2-hydroxyphenyl)-5-trifluoromethyl-2H-benzimidazol-2-one (0.2 g, 0.6 mmol) in concentrated sulfuric acid (2.5 ml) an ice cold solution of potassium nitrate (61 mg, 6 mmol) in concentrated sulfuric acid (1 ml) was added under stirring on an ice bath. The reaction mixture was poured onto ice (30 g) and the crude product was collected by filtration and recrystallized from ethanol:water 2:1 yielding the title compound as yellow crystals, M.p. 250°C (dec.).

The following compounds were prepared in a similar manner.

1,3-dihydro-1-(5-chloro-3-nitro-2-hydroxyphenyl)-5-trifluoromethyl-6-nitro-2H-benzimidazol-2-one, yellow crystals, M.p. 283-284°C, using two equivalents of potassium nitrate.

1,3-dihydro-1-(3,5-dichloro-2-hydroxyphenyl)-5,6-dinitro-2H-benzimidazol-2-one, yellow crystals, M.p. 300-303°C, using two equivalents of potassium nitrate.

1,3-dihydro-1-(5-nitro-2-hydroxyphenyl)-5-trifluoromethyl-6-nitro-2H-benzimidazole-2-one, yellow crystals, M.p. 285°C (decomp.), using two equivalents of potassium nitrate.

### EXAMPLE 18

### Compounds of Invention

1,3-dihydro-1-(5-chloro-2-hydroxyphenyl)-3-amino-5-trifluoromethyl-2H-benzimidazol-2-one.

To a solution of 1,3-dihydro-1-(5-chloro-2-hydroxyphenyl)-5-trifluoromethyl-2H-benzimidazol-2-one (0.2 g, 0.6 mmol) in absolute N,N-dimethylformamide (5 ml) potassium hydroxide (2 pellets) and hydroxylamine-*o*-sulfonic acid (0.16 g, 1.3 mmol) were added at room temperature. The reaction was stirred over night, water was added (20 ml) and the mixture was acidified with 4 M hydrochloric acid. The crude crystalline product was collected by filtration and subjected to column chromatography using methylene chloride:methanol (19:1) as eluent. The fractions containing the product were concentrated in vacuo yielding the title compound as white crystals, M.p. 228-230°C.

## Claims (Claims for the following Contracting State(s): AT, BE, CH, DE, DK, FR, GB, IT, LI, LU, NL, SE)

1. A compound having the formula 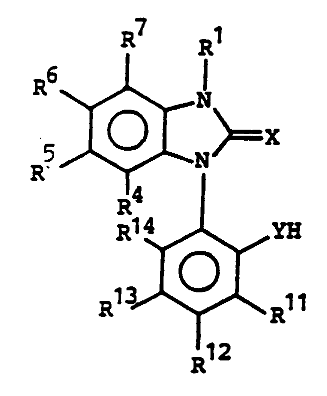 wherein R¹ is hydrogen, NH₂ or C₁₋₆-alkyl which may be branched; X is 0, S or NCN; Y is 0 or S; R⁴, R⁵, R⁶ and R⁷ each independently are hydrogen, halogen, CF₃, NO₂, NH₂, OH, C₁₋₆-alkoxy, C(=0)-phenyl or SO₂NR^{I}R^{II} wherein R^{I} and R^{II} independently are hydrogen or C₁₋₆-alkyl; R¹¹ is hydrogen, halogen, NO₂ or SO₂NR^{I}R^{II} wherein R¹ and R¹¹ independently are hydrogen or C₁₋₆-alkyl; R¹³ is hydrogen, halogen, phenyl, CF₃ or NO₂; R¹² is hydrogen or together with R¹³ forms a C₄₋₇-carbocyclic ring which may be aromatic or partially saturated; R¹⁴ is hydrogen or together with R¹³ forms a C₄₋₇-carbocyclic ring which may be aromatic or partially saturated.

2. A compound of claim 1 which is 5-trifluoromethyl-2,3-dihydro-1-(5-chloro-2-hydroxyphenyl)-1H-2-oxo-benzmidazole.

3. A compound of claim 1 which is 5-trifluoromethyl-2,3-dihydro-1-(5-phenyl-2-hydroxyphenyl)-1H-2-oxo-benzimidazole.

4. A compound of claim 1 which is 5-trifluoromethyl-6-nitro-2,3-dihydro-1-(3-nitro-5-chloro-2-hydroxyphenyl)-1H-2-oxo-benzimidazole.

5. A pharmaceutical composition comprising a therapeutically-effective amount of a compound of claim 1 together with a pharmaceutically-acceptable carrier.

6. A method of preparing a compound of claim 1 which comprises the step of reacting a compound having the formula 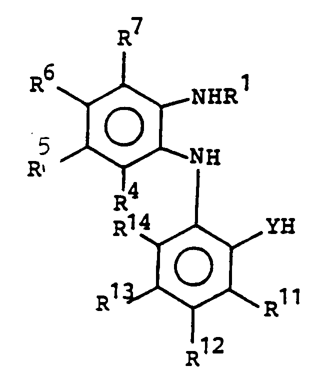 wherein R¹, R⁴, R⁵, R⁶, R⁷, Y, R¹¹, R¹², R¹³ and R¹⁴ have the meanings set forth in claim 1, with phosgene, thiocarbonyl diimidazol or N-cyanoimido-S,S'-dimethyldithiocarbonate.

7. Use of a compound of claim 1 for the manufacture of a medicament for the treatment of a disorder of an animal body, including human, said disorder being responsive to the opening of potassium channels.

8. The use according to claim 7, wherein said disorder is hypertension, asthma, ischemia or convulsions.

## Claims (Claims for the following Contracting State(s): ES)

1. A method of preparing a compound having the formula 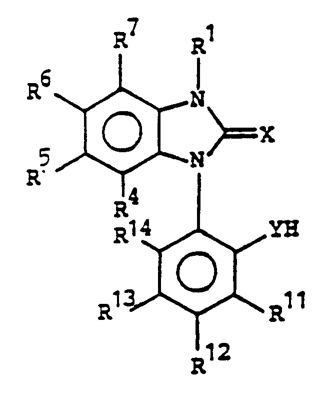 wherein R¹ is hydrogen, NH₂ or C₁₋₆-alkyl which may be branched; X is 0, S or NCN; Y is 0 or S; R⁴, R⁵, R⁶ and R⁷ each independently are hydrogen, halogen, CF₃, NO₂, NH₂, OH, C₁₋₆-alkoxy, C(=0)-phenyl or SO₂NR^{I}R^{II} wherein R^{I} and R^{II} independently are hydrogen or C₁₋₆-alkyl; R¹¹ is hydrogen, halogen, NO₂ or SO₂NR^{I}R^{II} wherein R¹ and R¹¹ independently are hydrogen or C₁₋₆-alkyl; R¹³ is hydrogen, halogen, phenyl, CF₃ or NO₂; R¹² is hydrogen or together with R¹³ forms a C₄₋₇-carbocyclic ring which may be aromatic or partially saturated; R¹⁴ is hydrogen or together with R¹³ forms a C₄₋₇-carbocyclic ring which may be aromatic or partially saturated,
which comprises the step of reacting a compound having the formula 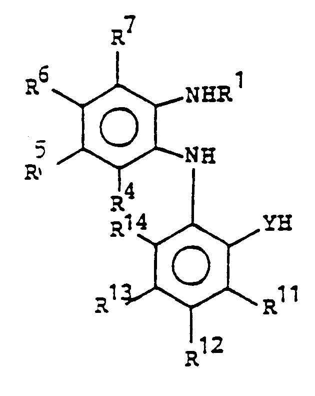 wherein R¹, R⁴, R⁵, R⁶, R⁷, Y, R¹¹, R¹², R¹³ and R¹⁴ have the meanings set forth above, with phosgene, thiocarbonyl diimidazol or N-cyanoimido-S,S'-dimethyldithiocarbonate.

2. The method of claim 1 wherein the obtained compound is 5-trifluoromethyl-2,3-dihydro-1-(5-chloro-2-hydroxyphenyl)-1H-2-oxo-benzmidazole.

3. The method of claim 1 wherein the obtained compound is 5-trifluoromethyl-2,3-dihydro-1-(5-phenyl-2-hydroxyphenyl)-1H-2-oxo-benzimidazole.

4. The method of claim 1 wherein the obtained compound is 5-trifluoromethyl-6-nitro-2,3-dihydro-1-(3-nitro-5-chloro-2-hydroxyphenyl)-1H-2-oxo-benzimidazole.

5. A process for preparing a pharmaceutical composition comprising mixing a therapeutically-effective amount of a compound prepared according to claim 1 with a pharmaceutically-acceptable carrier.

6. Use of a compound prepared according to claim 1 for the manufacture of a medicament for the treatment of a disorder of an animal body, including human, said disorder being responsive to the opening of potassium channels.

7. The use according to claim 6, wherein said disorder is hypertension, asthma, ischemia or convulsions.

## Claims (Claims for the following Contracting State(s): GR)

1. A compound having the formula 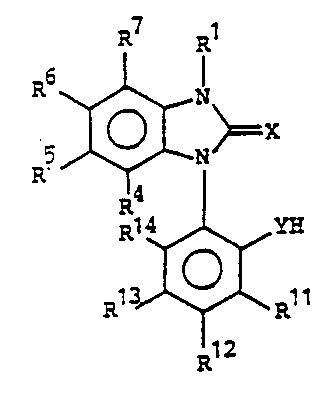 wherein R¹ is hydrogen, NH₂ or C₁₋₆-alkyl which may be branched; X is 0, S or NCN; Y is 0 or S; R⁴, R⁵, R⁶ and R⁷ each independently are hydrogen, halogen, CF₃, NO₂, NH₂, OH, C₁₋₆-alkoxy, C(=0)-phenyl or SO₂NR^{I}R^{II} wherein R^{I} and R^{II} independently are hydrogen or C₁₋₆-alkyl; R¹¹ is hydrogen, halogen, NO₂ or SO₂NR^{I}R^{II} wherein R¹ and R¹¹ independently are hydrogen or C₁₋₆-alkyl; R¹³ is hydrogen, halogen, phenyl, CF₃ or NO₂; R¹² is hydrogen or together with R¹³ forms a C₄₋₇-carbocyclic ring which may be aromatic or partially saturated; R¹⁴ is hydrogen or together with R¹³ forms a C₄₋₇-carbocyclic ring which may be aromatic or partially saturated.

2. A compound of claim 1 which is 5-trifluoromethyl-2,3-dihydro-1-(5-chloro-2-hydroxyphenyl)-1H-2-oxo-benzmidazole.

3. A compound of claim 1 which is 5-trifluoromethyl-2,3-dihydro-1-(5-phenyl-2-hydroxyphenyl)-1H-2-oxo-benzimidazole.

4. A compound of claim 1 which is 5-trifluoromethyl-6-nitro-2,3-dihydro-1-(3-nitro-5-chloro-2-hydroxyphenyl)-1H-2-oxo-benzimidazole.

5. A process for preparing a pharmaceutical composition which comprises mixing a therapeutically-effective amount of a compound of claim 1 with a pharmaceutically-acceptable carrier.

6. A method of preparing a compound of claim 1 which comprises the step of reacting a compound having the formula 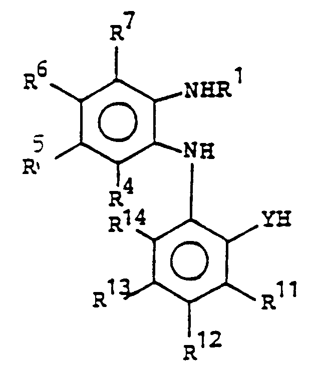 wherein R¹, R⁴, R⁵, R⁶, R⁷, Y, R¹¹, R¹², R¹³ and R¹⁴ have the meanings set forth in claim 1, with phosgene, thiocarbonyl diimidazol or N-cyanoimido-S,S'-dimethyldithiocarbonate.

7. Use of a compound of claim 1 for the manufacture of a medicament for the treatment of a disorder of an animal body, including human, said disorder being responsive to the opening of potassium channels.

8. The use according to claim 7, wherein said disorder is hypertension, asthma, ischemia or convulsions.

## Patentansprüche (Patentansprüche für folgende(n) Vertragsstaat(en): AT, BE, CH, DE, DK, FR, GB, IT, LI, LU, NL, SE)

1. Verbindung der Formel 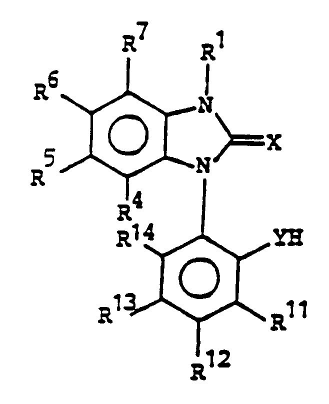 worin R¹ Wasserstoff, NH₂ oder C₁₋₆-Alkyl ist, das verzweigt sein kann; X O, S oder NCN ist; Y O oder S ist; R⁴, R⁵, R⁶ und R⁷ jeweils unabhängig voneinander Wasserstoff, Halogen, CF₃, NO₂, NH₂, OH, C₁₋₆-Alkoxy, C(=O)-Phenyl oder SO₂NR^{I}R^{II} sind, wobei R^{I} und R^{II} unabhängig voneinander Wasserstoff oder C₁₋₆-Alkyl sind; R¹¹ Wasserstoff, Halogen, NO₂ oder SO₂NR^{I}R^{II} ist, wobei R^{I} und R^{II} unabhängig voneinander Wasserstoff oder C₁₋₆-Alkyl sind; R¹³ Wasserstoff, Halogen, Phenyl, CF₃ oder NO₂ ist; R¹² Wasserstoff ist oder zusammen mit R¹³ einen C₄₋₇-carbocyclischen Ring bildet, der aromatisch oder teilweise gesättigt sein kann; R¹⁴ Wasserstoff ist oder zusammen mit R¹³ einen C₄₋₇-carbocyclischen Ring bildet, der aromatisch oder teilweise gesättigt sein kann.

2. Verbindung nach Anspruch 1, die 5-Trifluormethyl-2,3-dihydro-1-(5-chlor-2-hydroxyphenyl)-1H-2-oxo-benzimidazol ist.

3. Verbindung nach Anspruch 1, die 5-Triflrnormethyl-2,3-dihydro-1-(5-phenyl-2-hydroxyphenyl)-1H-2-oxo-benzimidazol ist.

4. Verbindung nach Anspruch 1, die 5-Trifluormethyl-6-nitro-2,3-dihydro-1-(3-nitro-5-chlor-2-hydroxyphenyl)-1H-2-oxo-benzimidazol ist.

5. Pharmazeutische Zusammensetzung, umfassend eine therapeutisch wirksame Menge einer Verbindung nach Anspruch 1, zusammen mit einem pharmazeutisch annehmbaren Träger.

6. Verfahren zum Herstellen einer Verbindung nach Anspruch 1, welches die Stufe des Reagierenlassens einer Verbindung der Formel 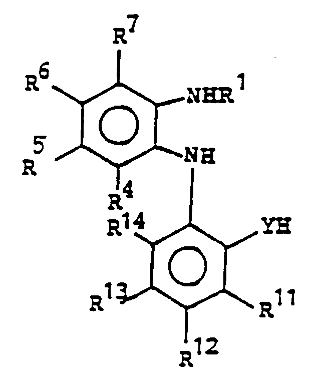 worin R¹, R⁴, R⁵, R⁶, R⁷, Y, R¹¹, R¹², R¹³ und R¹⁴ die in Anspruch 1 angegebenen Bedeutungen haben, mit Phosgen, Thiocarbonyldiimidazol oder N-Cyanoimido-S,S'-dimethyl-dithiocarbonat umfaßt.

7. Verwendung einer Verbindung nach Anspruch 1 für die Herstellung eines Medikaments für die Behandlung einer Krankheit eines Tierkörpers, einschließlich Mensch, wobei die Krankheit auf das öffnen der Kaliumkanäle reagiert.

8. Verwendung nach Anspruch 7, wobei die Krankheit Hypertonie, Asthma, Ischämie oder Konvulsionen ist.

## Patentansprüche (Patentansprüche für folgende(n) Vertragsstaat(en): ES)

1. Verfahren zum Herstellen einer Verbindung der Formel 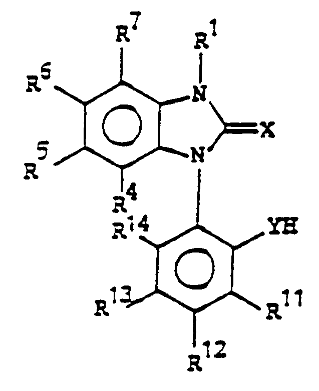 worin R¹ Wasserstoff, NH₂ oder C₁₋₆-Alkyl ist, das verzweigt sein kann; X O, S oder NCN ist; Y O oder S ist; R⁴, R⁵, R⁶ und R⁷ jeweils unabhängig voneinander Wasserstoff, Halogen, CF₃, NO₂, NH₂, OH, C₁₋₆-Alkoxy, C(=O)-Phenyl oder SO₂NR^{I}R^{II} sind, wobei R^{I} und R^{II} unabhängig voneinander Wasserstoff oder C₁₋₆-Alkyl sind; R¹¹ Wasserstoff, Halogen, NO₂ oder so₂NR^{I}R^{II} ist, wobei R^{I} und R^{II} unabhängig voneinander Wasserstoff oder C₁₋₆-Alkyl sind; R¹³ Wasserstoff, Halogen, Phenyl, CF₃ oder NO₂ ist; R¹² Wasserstoff ist oder zusammen mit R¹³ einen C₄₋₇-carbocyclischen Ring bildet, der aromatisch oder teilweise gesättigt sein kann; R¹⁴ Wasserstoff ist oder zusammen mit R¹³ einen C₄₋₇-carbocyclischen Ring bildet, der aromatisch oder teilweise gesättigt sein kann, welches die Stufe des Reagierenlassens einer Verbindung der Formel 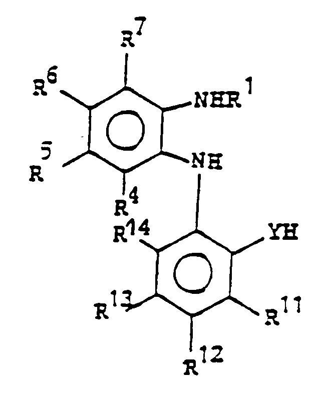 worin R¹, R⁴, R⁵, R⁶, R⁷, Y, R¹¹, R¹², R¹³ und R¹⁴ die vorstehenden Bedeutungen haben, mit Phosgen, Thiocarbonyldiimidazol oder N-Cyanoimido-S,S'-dimethyl-dithiocarbonat umfaßt.

2. Verfahren nach nach Anspruch 1, wobei die erhaltene Verbindung 5-Trifluormethyl-2,3-dihydro-1-(5-chlor-2-hydroxyphenyl)-1H-2-oxo-benzimidazol ist.

3. Verfahren nach Anspruch 1, wobei die erhaltene Verbindung 5-Trifluormethyl-2,3-dihydro-1-(5-phenyl-2-hydroxyphenyl)-1H-2-oxo-benzimidazol ist.

4. Verfahren nach Anspruch 1, wobei die erhaltene Verbindung 5-Trifluormethyl-6-nitro-2,3-dihydro-1-(3-nitro-5-chlor-2-hydroxyphenyl)-1H-2-oxo-benzimidazol ist.

5. Verfahren zum Herstellen einer pharmazeutischen Zusammensetzung, umfassend das Mischen einer therapeutisch wirksamen Menge einer Verbindung, hergestellt nach Anspruch 1, mit einem pharmazeutisch annehmbaren Träger.

6. Verwendung einer Verbindung, hergestellt nach Anspruch 1, für die Herstellung eines Medikaments für die Behandlung einer Krankheit eines Tierkörpers, einschließlich Mensch, wobei die Krankheit auf das Öffnen der Kaliumkanäle reagiert.

7. Verwendung nach Anspruch 6, wobei die Krankheit Hypertonie, Asthma, Ischämie oder Konvulsionen ist.

## Patentansprüche (Patentansprüche für folgende(n) Vertragsstaat(en): GR)

1. Verbindung der Formel 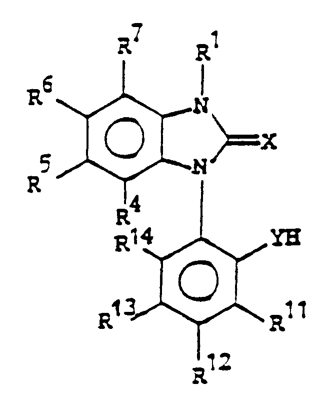 worin R¹ Wasserstoff, NH₂ oder C₁₋₆-Alkyl ist, das verzweigt sein kann; X O, S oder NCN ist; Y O oder S ist; R⁴, R⁵, R⁶ und R⁷ jeweils unabhängig voneinander Wasserstoff, Halogen, CF₃, NO₂, NH₂, OH, C₁₋₆-Alkoxy, C(=O)-Phenyl oder SO₂NR^{I}R^{II} sind, wobei R^{I} und R^{II} unabhängig voneinander Wasserstoff oder C₁₋₆-Alkyl sind; R¹¹ Wasserstoff, Halogen, NO₂ oder SO₂NR^{I}R^{II} ist, wobei R^{I} und R^{II} unabhängig voneinander Wasserstoff oder C₁₋₆-Alkyl sind; R¹³ Wasserstoff, Halogen, Phenyl, CF₃ oder NO₂ ist; R¹² Wasserstoff ist oder zusammen mit R¹³ einen C₄₋₇-carbocyclischen Ring bildet, der aromatisch oder teilweise gesättigt sein kann; R¹⁴ Wasserstoff ist oder zusammen mit R¹³ einen C₄₋₇-carbocyclischen Ring bildet, der aromatisch oder teilweise gesättigt sein kann.

2. Verbindung nach Anspruch 1, die 5-Trifluormethyl-2,3-dihydro-1-(5-chlor-2-hydroxyphenyl)-1H-2-oxo-benzimidazol ist.

3. Verbindung nach Anspruch 1, die 5-Trifluormethyl-2,3-dihydro-1-(5-phenyl-2-hydroxyphenyl)-1H-2-oxo-benzimidazol ist.

4. Verbindung nach Anspruch 1, die 5-Trifluormethyl-6-nitro-2,3-dihydro-1-(3-nitro-5-chlor-2-hydroxyphenyl)-1H-2-oxo-benzimidazol ist.

5. Verfahren zum Herstellen einer pharmazeutischen Zusammensetzung, welches das Mischen einer therapeutisch wirksamen Menge einer Verbindung nach Anspruch 1 mit einem pharmazeutisch annehmbaren Träger umfaßt.

6. Verfahren zum Herstellen einer Verbindung nach Anspruch 1, welches die Stufe des Reagierenlassens einer Verbindung der Formel 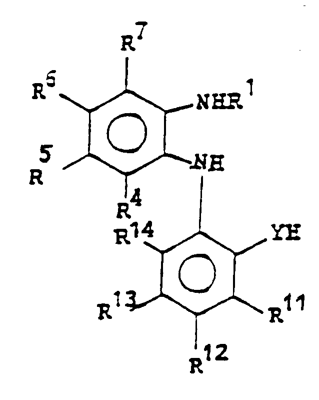 worin R¹, R⁴, R⁵, R⁶, R⁷, Y, R¹¹, R¹², R¹³ und R¹⁴ die in Anspruch 1 angegebenen Bedeutungen haben, mit Phosgen, Thiocarbonyldiimidazol oder N-Cyanoimido-S,S'-dimethyl-dithiocarbonat umfaßt.

7. Verwendung einer Verbindung nach Anspruch 1 für die Herstellung eines Medikaments für die Behandlung einer Krankheit eines Tierkörpers, einschließlich Mensch, wobei die Krankheit auf das Öffnen der Kaliumkanäle reagiert.

8. Verwendung nach Anspruch 7, wobei die Krankheit Hypertonie, Asthma, Ischämie oder Konvulsionen ist.

## Revendications (Revendications pour l'(les) Etat(s) contractant(s) suivant(s): AT, BE, CH, DE, DK, FR, GB, IT, LI, LU, NL, SE)

1. Composé ayant la formule 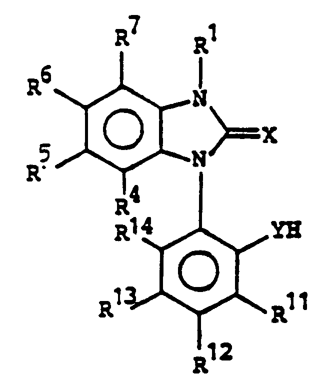 où R¹ est de l'hydrogène, NH₂ ou un groupe alkyle en C₁ à C₆ qui peut être ramifié ; X est 0, S ou NCN ; Y est O ou S ; R⁴, R⁵, R⁶ et R⁷ sont chacun indépendamment de l'hydrogène, un halogène, CF₃, NO₂, NH₂, OH, un groupe alcoxy en C₁ à C₆, un groupe C(=O)-phényle ou SO₂NR^{I}R^{II} où R^{I} et R^{II} sont indépendamment de l'hydrogène ou un groupe alkyle en C₁ à C₆ ; R¹¹ est de l'hydrogène, un halogène, NO₂ ou SO₂NR^{I}R^{II} où R^{I} et R^{II} sont indépendamment de l'hydrogène ou un groupe alkyle en C₁ à C₆ ; R¹³ est de l'hydrogène, un halogène, un groupe phényle, CF₃ ou NO₂ ; R¹² est de l'hydrogène ou, avec R¹³, forme un cycle carbocyclique en C₄ à C₇ qui peut être aromatique ou partiellement saturé ; R¹⁴ est de l'hydrogène ou, avec R¹³, forme un cycle carbocyclique en C₄ à C₇ qui peut être aromatique ou partiellement saturé.

2. Composé selon la revendication 1, qui est le 5-trifluorométhyl-2,3-dihydro-1-(5-chloro-2-hydroxyphényl)-1H-2-oxo-benzimidazole.

3. Composé selon la revendication 1, qui est le 5-trifluorométhyl-2,3-dihydro-1-(5-phényl-2-hydroxyphényl)-1H-2-oxo-benzimidazole.

4. Composé selon la revendication 1, qui est le 5-trifluorométhyl-6-nitro-2,3-dihydro-1-(3-nitro-5-chloro-2-hydroxyphényl)-1H-2-oxo-benzimidazole.

5. Composition pharmaceutique comprenant une quantité thérapeutiquement efficace d'un composé selon la revendication 1 avec un support pharmaceutiquement acceptable.

6. Procédé de préparation d'un composé selon la revendication 1, qui comprend l'étape de réaction d'un composé ayant la formule 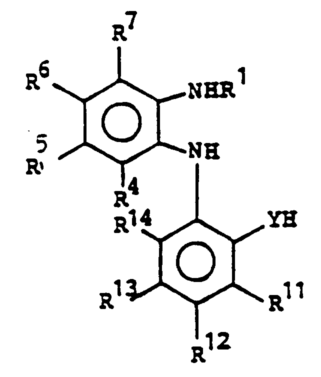 où R¹, R⁴, R⁵, R⁶, R⁷, Y, R¹¹, R¹², R¹³ et R¹⁴ ont les significations exposées dans la revendication 1, avec du phosgène, du thiocarbonyldiimidazole ou du dithiocarbonate de N-cyanoimido-S,S'-diméthyle.

7. Utilisation d'un composé selon la revendication 1 pour la fabrication d'un médicament pour le traitement d'un trouble de l'organisme d'un animal, y compris l'homme, ledit trouble étant sensible à l'ouverture des canaux à potassium.

8. Utilisation selon la revendication 7, dans laquelle ledit trouble est l'hypertension, l'asthme, l'ischémie ou les convulsions.

## Revendications (Revendications pour l'(les) Etat(s) contractant(s) suivant(s): ES)

1. Procédé de préparation d'un composé ayant la formule 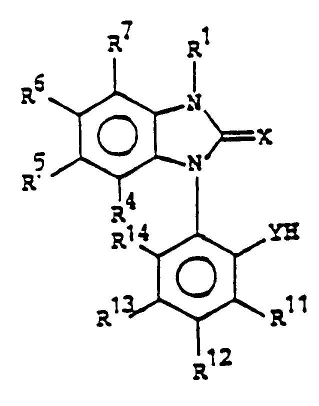 où R¹ est de l'hydrogène, NH₂ ou un groupe alkyle en C₁ à C₆ qui peut être ramifié ; X est 0, S ou NCN ; Y est O ou S ; R⁴, R⁵, R⁶ et R⁷ sont chacun indépendamment de l'hydrogène, un halogène, CF₃, NO₂, NH₂, OH, un groupe alcoxy en C₁ à C₆, un groupe C(=O)-phényle ou SO₂NR^{I}R^{II} où R^{I} et R^{II} sont indépendamment de l'hydrogène ou un groupe alkyle en C₁ à C₆ ; R¹¹ est de l'hydrogène, un halogène, NO₂ ou SO₂NR^{I}R^{II} où R^{I} et R^{II} sont indépendamment de l'hydrogène ou un groupe alkyle en C₁ à C₆ ; R¹³ est de l'hydrogène, un halogène, un groupe phényle, CF₃ ou NO₂ ; R¹² est de l'hydrogène ou, avec R¹³, forme un cycle carbocyclique en C₄ à C₇ qui peut être aromatique ou partiellement saturé ; R¹⁴ est de l'hydrogène ou, avec R¹³, forme un cycle carbocyclique en C₄ à C₇ qui peut être aromatique ou partiellement saturé, qui comprend l'étape de réaction d'un composé ayant la formule 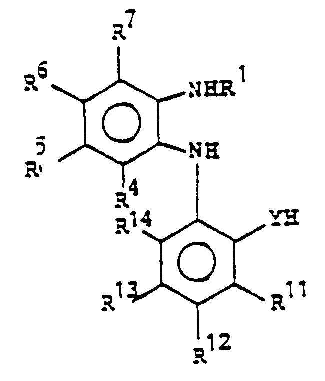 où R¹, R⁴, R⁵, R⁶, R⁷, Y, R¹¹, R¹², R¹³ et R¹⁴ ont les significations exposées ci-dessus, avec du phosgène, du thiocarbonyldiimidazole ou du dithiocarbonate de N-cyanoimido-S,S'-diméthyle.

2. Procédé selon la revendication 1, dans lequel le composé obtenu est le 5-trifluorométhyl-2,3-dihydro-1-(5-chloro-2-hydroxyphényl)-1H-2-oxo-benzimidazole.

3. Procédé selon la revendication 1, dans lequel le composé obtenu est le 5-trifluorométhyl-2,3-dihydro-1-(5-phényl-2-hydroxyphényl)-1H-2-oxo-benzimidazole.

4. Procédé selon la revendication 1, dans lequel le composé obtenu est le 5-trifluorométhyl-6-nitro-2,3-dihydro-1-(3-nitro-5-chloro-2-hydroxyphényl)-1H-2-oxo-benzimidazole.

5. Procédé pour préparer une composition pharmaceutique comprenant le mélange d'une quantité thérapeutiquement efficace d'un composé préparé selon la revendication 1 avec un support pharmaceutiquement acceptable.

6. Utilisation d'un composé préparé selon la revendication 1 pour la fabrication d'un médicament pour le traitement d'un trouble de l'organisme d'un animal, y compris l'homme, ledit trouble étant sensible à l'ouverture des canaux à potassium.

7. Utilisation selon la revendication 6, dans laquelle ledit trouble est l'hypertension, l'asthme, l'ischémie ou les convulsions.

## Revendications (Revendications pour l'(les) Etat(s) contractant(s) suivant(s): GR)

1. Composé ayant la formule 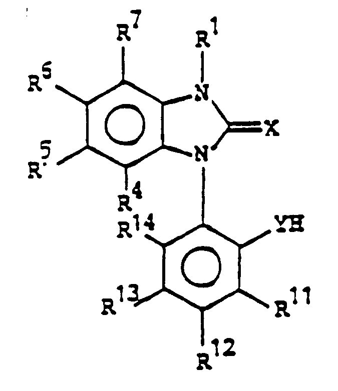 où R¹ est de l'hydrogène, NH₂ ou un groupe alkyle en C₁ à C₆ qui peut être ramifié ; X est 0, S ou NCN ; Y est O ou S ; R⁴, R⁵, R⁶ et R⁷ sont chacun indépendamment de l'hydrogène, un halogène, CF₃, NO₂, NH₂, OH, un groupe alcoxy en C₁ à C₆, un groupe C(=O)-phényle ou SO₂NR^{I}R^{II} où R^{I} et R^{II} sont indépendamment de l'hydrogène ou un groupe alkyle en C₁ à C₆ ; R¹¹ est de l'hydrogène, un halogène, NO₂ ou SO₂NR^{I}R^{II} où R^{I} et R^{II} sont indépendamment de l'hydrogène ou un groupe alkyle en C₁ à C₆ ; R¹³ est de l'hydrogène, un halogène, un groupe phényle, CF₃ ou NO₂ ; R¹² est de l'hydrogène ou, avec R¹³, forme un cycle carbocyclique en C₄ à C₇ qui peut être aromatique ou partiellement saturé ; R¹⁴ est de l'hydrogène ou, avec R¹³, forme un cycle carbocyclique en C₄ à C₇ qui peut être aromatique ou partiellement saturé.

2. Composé selon la revendication 1, qui est le 5-trifluorométhyl-2,3-dihydro-1-(5-chloro-2-hydroxyphényl)-1H-2-oxo-benzimidazole.

3. Composé selon la revendication 1, qui est le 5-trifluorométhyl-2,3-dihydro-1-(5-phényl-2-hydroxyphényl)-1H-2-oxo-benzimidazole.

4. Composé selon la revendication 1, qui est le 5-trifluorométhyl-6-nitro-2,3-dihydro-1-(3-nitro-5-chloro-2-hydroxyphényl)-1H-2-oxo-benzimidazole.

5. Procédé pour préparer une composition pharmaceutique comprenant le mélange d'une quantité thérapeutiquement efficace d'un composé selon la revendication 1 avec un support pharmaceutiquement acceptable.

6. Procédé de préparation d'un composé selon la revendication 1, qui comprend l'étape de réaction d'un composé ayant la formule 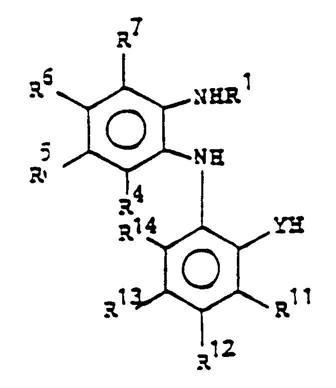 où R¹, R⁴, R⁵, R⁶, R⁷, Y, R¹¹, R¹², R¹³ et R¹⁴ ont les significations exposées dans la revendication 1, avec du phosgène, du thiocarbonyldiimidazole ou du dithiocarbonate de N-cyanoimido-S,S'-diméthyle.

7. Utilisation d'un composé selon la revendication 1 pour la fabrication d'un médicament pour le traitement d'un trouble de l'organisme d'un animal, y compris l'homme, ledit trouble étant sensible à l'ouverture des canaux à potassium.

8. Utilisation selon la revendication 7, dans laquelle ledit trouble est l'hypertension, l'asthme, l'ischémie ou les convulsions.
